Europäisches Patentamt

(19) European Patent Office

Office européen des brevets

(11) Publication number: **0 043 259**

**A1**

(12) **EUROPEAN PATENT APPLICATION**

(21) Application number: **81302904.8**

(22) Date of filing: **26.06.81**

(51) Int. Cl.³: **C 12 P 7/06**

(30) Priority: **27.06.80 GB 8021095**
**29.09.80 GB 8031346**

(43) Date of publication of application:
**06.01.82 Bulletin 82/1**

(84) Designated Contracting States:
**AT BE CH DE FR IT LI LU NL SE**

(71) Applicant: **A.G.(PATENTS) LIMITED**
**156 St.John Street**
**London, ECIP 1AR(GB)**

(72) Inventor: **Fricker, Richard**
**129 Whitaker Road**
**Derby(GB)**

(74) Representative: **Holmes, Michael John et al,**
**Frank B. Dehn & Co. European Patent Attorneys Imperial**
**House 15-19 Kingsway**
**London, WC2B 6UZ,(GB)**

(54) A method and apparatus for fermentation.

㊷ A method for fermentation in which carbohydrate solution is discontinuously fed to a fermentation zone containing yeast whereby the carbohydrate is fermented to ethanol, a proportion of the fermenting liquid passing to a pressurised settling tank, yeast depleted liquid being withdrawn from the upper part of the settling tank and yeast enriched liquid being withdrawn from the lower part of said tank and returned to the fermentation zone, a proportion of yeast being withdrawn without being returned to the fermentation zone, said proportion being such that the concentration of yeast in the fermentation zone is substantially constant, and the pressure within the settling tank is sufficient to prevent the formation of gaseous carbon dioxide. The method enables rapid continuous fermentation to take place using high concentrations of yeast. Apparatus for operating the method is described.

EP 0 043 259 A1

0043259

A method and apparatus for fermentation

This invention relates to a novel method of fermentation and apparatus therefor.

The fermentation of carbohydrate to yield ethanol for use as a fuel is, to some extent, free from many of the restraints of traditional beer production created by the requirement for a consistent and acceptable flavour. There is now a demand for a rapid and efficient fermentation process for the production of ethanol for distillation. The well established fermentation methods using yeast have commonly tended to produce relatively large amounts of yeast as a by-product, due to growth of yeast during fermentation. Since the yeast uses carbohydrate as a nutrient, such growth inevitably leads to a reduction in the efficiency of carbohydrate conversion. The term "carbohydrate" as used herein means carbohydrates which are fermentable by yeast to form ethanol.

One method of minimising carbohydrate losses due to yeast growth is to re-cycle the yeast. This requires separation of yeast from the fermented liquor and in many cases is a time-consuming and occasionally troublesome process.

In our British Patent Specification No. 2059988A we have described a method and apparatus for continuous fermentation in which yeast-containing fermented liquid is continuously drawn off and passed through a settling tank in order to provide a yeast enriched stream for re-cycling, the settling tank operating under pressure in order to avoid liberation of gaseous carbon dioxide and so increase the buoyancy of the yeast. We have now found that such a method and apparatus has advantages which extend to batch and semi-continuous fermentation as well as continuous operation.

Our United Kingdom Patent Specification No. 2059988A thus describes a simple method of rapid continuous fermentation which uses high concentrations of yeast but overcomes some of the previous problems of prior art continuous fermentation systems.  In this method, a carbohydrate solution is passed through a fermentation zone containing suspended yeast at relatively high concentration, the yeast and carbohydrate in this zone being substantially homogeneously distributed, and a yeast-laden alcohol solution passes to a settling zone from which yeast enriched liquid is returned to the fermenter while yeast depleted liquid is drawn off e.g. to a still.  In order to maintain the yeast population in the fermenter substantially constant, some yeast is withdrawn without being returned to the fermentation zone and, most simply, where the alcohol is required for distillation, this yeast is merely passed to the still.

When the yeast concentration in the fermentation zone is maintained at a very high level, rapid sedimentation in the settling zone is essential in order to handle the relatively large volumes of yeast being recycled.  Sedimentation rates depend not only on the flocculating characteristics of the yeast but also on the buoyancy created by any bubbles of carbon dioxide trapped on the yeast surface which are generated by the continuation of fermentation in the settling tank.  In previous continuous fermentation systems using a homogeneous fermentation zone and re-cycling of yeast separated by sedimentation, the settling tank has been operated substantially at atmospheric pressure, and since the solution issuing from the fermentation zone is normally saturated with carbon dioxide, the carbon dioxide generated by further fermentation forms gaseous bubbles.  Where relatively small yeast volumes have been concerned, this has not been a serious disadvantage, but in the continuous fermentation system described in United Kingdom Patent Specification No. 2059988A, where high yeast concentrations are envisaged, it is necessary to reduce buoyancy to a minimum. The solution adopted is to maintain a sufficient pressure in the settling tank to prevent the formation of gaseous carbon dioxide. In general, a pressure of at least about $1.3 \times 10^5 Nm^{-2}$ (1.3 bars) e.g. at least $2 \times 10^5 Nm^{-2}$ (2 bars) absolute is needed to ensure this.

Although the method and apparatus described in the above United Kingdom Patent Specification is concerned with continuous fermentation, as indicated above we have now found that the good sedimentation which may be achieved by the method has a general application also to batch and so-called "semi-continuous" fermentation processes.

According to one aspect of the present invention, therefore, there is provided a method of fermentation, in which a carbohydrate solution is discontinuously fed to a fermentation zone to provide a substantially homogeneous dispersion of yeast in carbohydrate solution, the carbohydrate thus being fermented to ethanol, wherein at least a proportion of the fermented liquid is passed to a pressurised settling tank, and a substantially equal volume of carbohydrate solution is fed in to replace said fermented liquid, yeast depleted liquid being withdrawn from the upper part of the settling tank and yeast enriched liquid being withdrawn from the lower part of said tank and returned to the fermentation zone, a proportion of yeast being withdrawn without being returned to the fermentation zone, said proportion being such that the quantity of yeast in the fermentation zone is substantially the same during each fermentation, the pressure within the settling vessel being sufficient to prevent the formation of any gaseous carbon dioxide.

If such a method was operated so that the proportion of fermented liquor removed from the fermentation zone at the end of the fermentation period contained an equivalent proportion of the yeast initially present, and if the yeast was not re-cycled, then a considerable amount of the incoming fresh carbohydrate would be required for the growth of yeast in the fermentation zone, at the expense of ethanol production. The mean rate of fermentation would also be reduced. In order therefore to maximise the utilisation of incoming fresh carbohydrate for ethanol production and to maintain the highest possible fermentation rate, the method includes a yeast settling and recycling system. It is therefore generally important to use a yeast strain which exhibits good flocculating properties so that the desired degree of sedimentation takes place in a relatively short period of time. One particularly suitable yeast strain is yeast number CBS 7072 (deposited 29 May 1980 at the Centraal Bureau voor Schimmelcultures (Yeast Division), Delft, Netherlands).

In order for the yeast to remain viable in the method of the invention, it is generally desirable not to allow an ethanol concentration of more than about 10% by volume to be reached by the end of fermentation; although it may be possible to allow a concentration up to about 12%, and in the case of some yeasts, up to about 17% by volume of ethanol to be reached.

On the other hand, it is economically desirable that the ethanol concentration should be high in order to minimise distillation heat requirements.  The concentration of ethanol is thus preferably greater than 6% by volume.

The method and apparatus of the invention may be used in batch fermentation, that is by substantially emptying the fermentation zone after fermentation is complete and refilling with fresh carbohydrate solution.  It will be appreciated that in batch processes, yeast obtained from the separation zone will not be recycled to the fermentation zone until the fermented liquid has all been removed and it is wished to begin the next batch fermentation.

Another possible method of operation is "semi-continuous"; thus, a batch of fermentable liquid may be fermented to completion and a fraction, for example one quarter, of the liquid volume, including suspended yeast may then be passed to the settling tank for removal of yeast from the product.  The fermentation zone may then be refilled and the fermentation continued while yeast from the settling tank may be returned to the fermentation zone.

The conversion of hexoses into ethanol by yeast inevitably produces one molecule of carbon dioxide (MW 44) for every molecule of ethanol (MW 45) so that the weight of carbohydrate converted is approximately twice the weight of ethanol produced.  If the percentage volume of liquid replaced after each fermentation period in semi-continuous operation is $V\%$ and the overall ethanol concentration at the end of fermentation is desired to be $A\%$ by weight, it can be seen that at the beginning of each fermentation period, after addition of fresh carbohydrate, the ethanol concentration has fallen to $[(100-V)/100].A\%$ and that this concentration needs to be raised by $(V/100).A\%$.

This requires a concentration of carbohydrate in the fermentation medium, at the start of fermentation, of twice that value, namely $(2V/100).A\%$. However, the concentration of carbohydrate in the feedstock necessary to produce such an overall concentration in the fermentation medium is related to the relative volumes of feedstock and fermentation medium, i.e. $V:100$. Consequently, the concentration of carbohydrate in the feedstock is simply $2A\%$ and is thus independent of the percentage replacement volume.

We have generally found it convenient in semi-continuous operation to remove and replace up to about 40%, desirably 10-30% and preferably about 25% of the volume of fermented liquor at the end of each cycle of operations. It is however possible for smaller replacement volumes to be used, so that the method may approach continuous operation. In this case it is generally desirable to use a separate settling tank, either situated within the fermentation vessel or separated therefrom, in order to allow the desired degree of separation of fermented liquor from yeast to take place. In general, relatively large percentage replacement volumes may be more convenient in that as compared with smaller replacement volumes, they reduce the number of handling operations required in fermenting a given volume of feedstock and, at the start of each fermentation period, the alcohol concentration may be reduced to a lower level, thereby reducing any adverse effects of the alcohol on the efficiency of the fermentation.

The carbohydrate concentration in the fermentation zone at the start of fermentation in semi-continuous operation, as can be seen from the foregoing, is related to the percentage increase in the concentration of ethanol to be produced during the fermentation period and can be represented using the above symbols, as A(2V/100). Thus, for example, when the final ethanol concentration is desired to be about 10% by weight and 25% by volume of liquid is replaced by the same volume of 20% carbohydrate solution, it is necessary to use a carbohydrate concentration in the fermentation zone at the beginning of the fermentation period of about 5% by weight.

Using a high concentration of yeast in the fermentation zone, e.g. 6 to 20%, conveniently 8 to 15% and preferably about 12% by weight of wet pressed yeast, it is possible to obtain short fermentation times, which may in some cases be as low as ½ hour although times of 1 to 3 hours are more usual.

According to a further feature of the invention there is provided an apparatus for fermentation comprising a fermentation vessel having a fermentation zone and means for conducting liquid discontinuously into said zone and out of said zone to a settling tank capable of withstanding an absolute internal pressure of at least about $1.3 \times 10^5 Nm^{-2}$ (1.3 bars), means being provided to conduct yeast-enriched liquid from the bottom of the settling tank back to the fermentation zone, there being provided means to lead off yeast-depleted liquid from the top of the settling tank and means to control the quantity of yeast removed without return to the fermentation zone.

The apparatus according to the invention is provided with means for discontinuously feeding liquid into and out of the fermentation zone. Such means may, for example, take the form of valves suitably situated in the respective lines. In semi-continuous operation, such valves may be controlled by a timing mechanism, so that liquid is withdrawn and fed in at fixed intervals, or control means may be related to the extent of fermentation, for example by monitoring the specific gravity.

The pressure within the settling tank will, in general, be above about $1.3 \times 10^5 Nm^{-2}$ (1.3 bars) absolute pressure and will preferably exceed $2 \times 10^5 Nm^{-2}$ (2 bars). In that increased pressures place large stress on the walls of the tank, the pressure will not normally be greater than is strictly necessary to keep carbon dioxide in solution and in general, pressures above about $6 \times 10^5 Nm^{-2}$ (6 bars) are not normally required. A pressure of about $2 \times 10^5 Nm^{-2}$ (2 bars) is generally sufficient. It will be appreciated that the hydrostatic head of liquid within the settling tank will create somewhat higher pressures at the bottom of the tank than at the top.

The settling tank may be separate from the fermentation vessel or may be situated within the fermentation vessel at a point at which the hydrostatic head of liquid above the tank is at all times sufficient to maintain the desired internal pressure.

Where the settling tank is a separate vessel, the apparatus may comprise a fermentation vessel having an inlet and an outlet spaced from said inlet, a liquid flowline leading from said outlet to a settling tank separate from said fermentation vessel, a liquid flowline leading from the bottom of the settling tank back to the fermentation vessel, there being provided means to move liquid from the fermentation vessel to the settling tank, means to return yeast-enriched liquid from the bottom of the settling tank to the fermentation vessel, means to lead off yeast-enriched liquid from the bottom of the settling tank without returning it to the fermentation vessel, means to lead off yeast-depleted liquid from the top of the settling tank and means to control the quantity of yeast-enriched liquid removed without return to the fermentation vessel.

It may be convenient to withdraw all the yeast enriched liquid from the bottom of such a separate settling tank and to divide this by a suitable valve system. The valve positions may, for example, be operated by a cyclic timer to regulate the time for which the yeasty liquid flows along each respective line. The system will be set to maintain the yeast concentration in the fermentation zone at a relatively high level, for example 6-15%, e.g. 10-14% by weight of wet pressed yeast.

Where the fermented product is required for distillation the yeasty liquid which is not recycled can simply be mixed with the yeast depleted liquid for passage to the still. The presence of yeast in the still liquor is not disadvantageous and a proportion of the spent liquid from the still can, indeed, be mixed with the initial carbohydrate solution to provide nutrient material for the fermentation, as well as conserving heat and reducing effluent.

The carbohydrate solution will normally be derived from natural cellulose or starch by enzymic or other hydrolysis or from molasses or other natural sugars. Further nutrients may be present to facilitate optimal performance of the yeast. As indicated above, a proportion of the spent liquid from the still may be added, thereby permitting recycling of unfermented carbohydrate and utilising the nitrogen content of the autolysed yeast. The carbohydrate solution will normally be aerated before entering the fermenter.

In order to promote efficient sedimentation of yeast in the settling tank, yeast activity may be suppressed by lowering the temperature, e.g. to about 10-15$^{\circ}$C. However, this may be unnecessary or even undesirable with some yeasts.

By utilising a high concentration of yeast in the fermentation zone, the rate of fermentation can be high and alcohol thereby produced particularly rapidly. However, removal of heat from the fermentation zone requires particular attention under these circumstances and while the fermentation zone could be provided with a cooling jacket for removal of heat, with internal agitation of the medium to ensure uniform temperature conditions, a more effective method of heat removal is to recirculate medium from the fermentation zone through a heat-exchanger. This technique also serves to agitate the fermentation medium to ensure substantially uniform conditions in the fermentation zone. It is convenient, therefore, to pump yeasty liquid from the fermentation zone through a cooled heat exchanger and back into the fermentation zone. This recirculation loop can advantageously be combined with the system for passing yeasty liquid to the settling tank. Where it is desired to cool the yeasty liquid entering the settling tank to promote sedimentation, it is advantageous to lead a proportion of the medium passing through the recirculation loop from a point downstream of the heat exchanger, through the settling tank and to return the yeast concentrate from the settling tank back into the recirculation loop at a point further downstream. If a constriction is provided in the recirculation loop between the junctions leading to and from the settling tank, the pump recirculating liquid through the recirculating loop may be used to drive liquid through the settling tank. Valves may be used to control the proportion of the recirculating liquid led off through the settling tank. If it is not desired to cool the liquid passing to the settling tank, the junctions leading to and from the settling tank may be upstream of the heat exchanger.

The rate of recirculation through the heat exchanger is determined mainly by the degree of agitation of the fermentation medium which is required. The heat load to be removed is determined by the rate of fermentation, and the inlet temperature of the heat exchanger is that of the fermentation medium in the fermentation zone, which should be maintained at its optimal value by adjusting the rate of coolant flow to the heat exchanger. The outlet temperature of the heat exchanger is thus determined largely by these factors and the size and cooling rate of the heat exchanger. The temperature of the liquid entering the settling vessel may be varied over a small range by adjusting the rate of re-circulation; nevertheless the temperature reduction achieved by the heat exchanger is normally quite sufficient to achieve the desired sedimentation of yeast.

Control of pH in the fermentation zone is conveniently effected by introducing acid or alkali into the recycling loop.

It is convenient to maintain the pH of the fermenting liquor at a relatively low value e.g. in the range 2.5 to 4 and preferably at about 3.5 However, the pH should generally not be too low as this may adversely affect both the rate of fermentation and the rate of sedimentation of yeast in the settling period. When the pH of the fermenting liquor is maintained in the above range, provided hygenic practices are rigorously observed, it is not always necessary to sterlise the incoming carbohydrate solution as the low pH will help to ensure that the growth of any undesired micro-organisms, such as bacteria, in the fermentation zone is inhibited. Similarly, when beginning a new fermentation, additional yeast from a yeast propagator may if required also be introduced into the recycling loop.

The cold liquid passing from the settling tank to the still may be used to cool the incoming carbohydrate solution, which may, in some cases, be held at a high temperature, e.g. $82^{o}C$, to maintain sterility, down to the fermentation temperature (which will usually be about $30^{o}C$) and will itself be heated e.g. up to above $77^{o}C$, thereby saving heat energy required for distillation. Similarly, a proportion of hot spent liquid from the still may, if required, be passed through a heat exchanger to heat the incoming water entering the carbohydrate preparation vessel, which in many cases will be maintained at a high temperature.

Where sedimentation rates are low, the cross-sectional area of the settling tank must be large in order to obtain adequate separation. It is apparent that the combination of requirements for pressure containment and large diameter of the settling vessel can lead to expensive structures.

These disadvantages may be eliminated by combining the settling tank into the fermentation vessel in such a way that the hydrostatic pressure in the fermentation vessel is used to pressurise the settling tank so that the common walls between the two vessels are not subjected to significant differential pressure and thus carry little or no pressure-induced load. As a result the thickness of the common walls can be substantially reduced with an attendant reduction in fabrication costs.

In the production of ethanol, the yeast concentration, fermentation and settling temperatures and the pressure in the settling tank may be in the same ranges as indicated above for apparatus using a separate settling tank. Adequate pressure can be achieved if the head of fermenting liquor above the settling tank is sufficiently great, e.g. about 3 metres or greater, e.g. from 10 metres up to about 25 metres.

When first operating the method of the invention it will generally be necessary to initiate a yeast propagation stage before ethanol production can commence. The purpose of such a stage being to grow the required quanitity of yeast for the fermentation process. Alternatively the required body of yeast could be separately produced in a yeast propagator. The yeast is first grown according to conventional practice, that is with sufficient carbohydrate (and, if necessary, minerals and nutrients) and in the presence of sufficient oxygen. In the propagation stage it is generally necessary to sterlise the carbohydrate feed solution to prevent the growth of extraneous micro-organisms.

When sufficient yeast has been grown in the fermentation zone, or sufficient yeast introduced thereto from a separate propagator, then the method of the invention may be started by filling the zone with feedstock carbohydrate solution and fermenting to completion. Whereafter the method of the invention may be operated as described above. In general, in order to maximise conversion of carbohydrate to ethanol, yeast growth during fermentation should be kept to a minimum. One benefit of using high yeast concentrations is that this tends to inhibit yeast growth.

It is also possible to effect semi-continuous fermentation without the use of a separate settling tank. In such a method of operation, carbohydrate solution is fermented and, instead of passing the fermented liquor to the separate settling tank, a settling period is provided for sedimentation of yeast under gravity in the fermentation zone between completion of fermentation and the removal of a proportion of the fermented liquor.

Thus, in another aspect, the invention provides a method of fermentation in which a carbohydrate solution is fermented to ethanol with a yeast, which method comprises the steps of a) passing carbohydrate solution to a fermentation zone containing yeast whereby the carbohydrate is fermented to ethanol; b) allowing the yeast to settle under gravity in the fermentation zone when fermentation is substantially complete; c) subsequently removing a proportion of fermented liquor substantially free of yeast suspended therein from the fermentation zone; and d) replacing the proportion of fermented liquor removed from the fermentation zone with fresh carbohydrate solution.

In this method of operation, when fermentation is finished, the fermented liquor is allowed to stand without any agitation whereupon the yeast will settle under gravity. Using a highly flocculent yeast, a settling time of about 3 hours may be sufficient and in some cases this may be reduced to as little as half an hour.

The shape and dimensions of the vessel used for fermentation and settling in such a semi-continuous method of operation is relatively unimportant. However, to ensure rapid and efficient sedimentation of yeast in the settling stage it may be convenient to employ a vessel having a large cross-sectional area in relation to its volume.

- 15 -

When using a single vessel in semi-continuous operation, this may advantageously be fitted with an articulated draw-off pipe internally supported by a float, such that fermented liquid may be decanted from a shallow depth below the liquid surface. By this means, withdrawal of the fermented liquid may commence very shortly after the start of the settling period and may continue throughout that period thereby minimising the required pumping rate and the size of the withdrawal pump.

Similarly to minimise the required carbohydrate feed rate, feeding may take place over most of the fermentation period, although it is desirable to allow at least ½ hour without feeding at the end of the fermentation period to ensure that fermentation is complete.

The method and apparatus according to the invention may be used, in fact, for many fermentation processes, in addition to processes primarily intended for alcohol (ethanol) production (both for potable and non-potable alcohol production) for example in antibiotic production.

Apparatus which may be used in the methods of the invention, is that described by way of illustration and with reference to Figures 1 to 7 in our United Kingdom Patent Specification No. 2059988A.

The invention will now be illustrated by the following non-limiting Examples:-

Example 1

An enclosed vertical cylindrical fermenting tank with shallow conical bottom as described in our U.K. Patent No. 2059988A, Figure 2, having a total capacity of 650 cubic metres, a diameter of 8 metres and a straight section height of 12 metres was fitted with an internal divider or hood in order to create separate zones for agitated fermentation and quiescent settling. The divider was positioned such that the height of fermenting liquid above its uppermost point was 10 metres. The fermenter was charged with 620 cubic metres of 20 weight percent glucose solution supplemented with nutrient materials to provide for controlled yeast growth and sufficient yeast of strain No. CBS 7072 of the Centraal Bureau voor Schimmelcultures was added to give a concentration of 12 volume percent pressed yeast. The tank was agitated by pump recirculation as shown in the above mentioned Figure 2 until fermentation was complete, sufficient air being injected into the recirculation loop to maintain the yeast in a viable condition. When fermentation had ceased a proportion of the vessel contents was withdrawn via conduit 25 after clarification by transit through the settling zone 23. This material was pumped to a holding tank for subsequent distillation. Immediately after withdrawal of the fermented product a fresh charge of 20 weight percent glucose solution was added to the fermenter and fermentation was again allowed to proceed. After completion of fermentation a proportion of the vessel contents was again withdrawn and the cycle was repeated continuously for a period of three weeks. In each cycle the yeast removed by the clarification process was retained within the fermentation zone by the combined action of the recirculation and resettling systems.

The proportion of fermenter contents removed was varied between 5 and 40%. With 25% withdrawal the total cycle time was six hours. With other quantities withdrawn the cycle time was extended/reduced in approximate proportion to the amount of the fermenter contents removed at each cycle.

Example 2

A vertical cylindrical fermenting tank of 720 gallons (3211 litres) capacity was charged with 580 gallons (2587 litres) of 20 percent by weight glucose solution supplemented with nutrient materials to provide for yeast growth. The dimensions of the tank were such as to give a ratio of height to diameter in the contained liquid of 1.3:1 800 lbs (363 kg) of pressed yeast of strain No. CBS 7072 of the Centraal Bureau voor Schimmelcultures was added and fermentation was allowed to proceed. The tank was agitated by means of a flow of air at a rate of 40 litres per minute applied through a perforated sparge pipe for the whole of the active fermentation period. When fermentation has ceased the agitating air was turned off and the yeast was allowed to settle to a level corresponding to three-quarters of the liquid volume present. The supernatant fermented liquor amounting to one-quarter of the volume present was then decanted from the vessel through an appropriately located side-arm and pumped to a holding tank prior to distillation. A fresh charge of 20 percent glucose solution was then added to the fermenter, the agitating air was turned on and fermentation was again allowed to proceed. After completion of fermentation the yeast was again allowed to settle and the supernatant liquid was removed. This cycle was repeated on a continuing basis for a period of two weeks.

The fermentation was found to take place over a period of four hours to completion, whilst the yeast settling operation occupied one hour. It was thus found possible to operate the system on a 6-hour repeating cycle giving a nominal residence time of 24 hours. The fermented product contained approximately 9.5 percent by weight ethanol and substantially no fermentable sugar.

It was found to be possible to agitate the vessel with carbon dioxide instead of air for three cycles out of the four, thus facilitating the collection of pure carbon dioxide uncontaminated with air for three quarters of the fermenting time.

CLAIMS:

1.  A method of fermentation, in which a carbohydrate solution is discontinuously fed to a fermentation zone to provide a substantially homogeneous dispersion of yeast in carbohydrate solution, the carbohydrate thus being fermented to ethanol, wherein at least a proportion of the fermented liquid is passed to a pressurised settling tank, and a substantially equal volume of carbohydrate solution is fed in to replace said fermented liquid, yeast depleted liquid being withdrawn from the upper part of the settling tank and yeast enriched liquid being withdrawn from the lower part of said tank and returned to the fermentation zone, a proportion of yeast being withdrawn without being returned to the fermentation zone, said proportion being such that the quantity of yeast in the fermentation zone is substantially the same during each fermentation, the pressure within the settling vessel being sufficient to prevent the formation of any gaseous carbon dioxide.

2.  A method according to claim 1 in which only a proportion of the fermented liquid is passed from the fermentation zone to the pressurised settling tank.

3.  A method according to claim 2 in which the proportion of fermented liquid passed from the fermentation zone to the pressurised settling tank is from 10 to 40% of the volume of fermented liquid in the fermentation zone.

4.  A method according to any one of the preceding claims in which the yeast depleted liquid is subjected to distillation.

5.  A method according to any one of the preceding claims in which the yeast concentration in the fermentation vessel is in the range 10-14% by weight of wet pressed yeast.

6.  A method according to any one of the preceding claims in which the carbohydrate solution is derived from natural cellulose or starch or from molasses or other natural sugars.

7.  A method according to any one of the preceding claims in which the fermentation vessel is cooled and agitated by passing medium from the fermentation vessel through a re-circulation loop containing a heat-exchanger, liquid being drawn off from said recirculation loop to the settling tank and yeast-enriched liquid being returned from the settling tank to the recirculation loop.

8.  A method according to any one of the preceding claims in which the pressure within the settling tank is at least about $1.3 \times 10^5 Nm^{-2}$ absolute.

9.  A method according to claim 8 in which the pressure in the settling tank is about $2 \times 10^5 Nm^{-2}$ absolute.

10. A method according to any one of the preceding claims in which the settling tank is situated within a fermentation vessel below a fermentation zone in said vessel, so that the hydrostatic pressure within the fermentation vessel creates the required pressure in the settling tank, while the common walls between
the fermentation zone and the settling tank are not
subjected to significant differential pressure.

11. An apparatus for fermentation comprising a
fermentation vessel having a fermentation zone and
means for conducting liquid discontinuously into said
zone and out of said zone to a settling tank which,
when separate from said fermentation vessel, is capable
of withstanding an absolute internal pressure of at
least about $1.3 \times 10^5 Nm^{-2}$, means being provided to
conduct yeast-enriched liquid from the bottom of the
settling tank back to the fermentation zone, there being
provided means to lead off yeast-depleted liquid from
the top of the settling tank and means to control the
quantity of yeast removed without return to the
fermentation zone.

12. An apparatus for fermentation which comprises
a combined fermentation/separation vessel having a
fermentation zone wherein fermentation of yeast and
carbohydrate solution may take place, said fermentation
zone being situated above a settling zone wherein at
least partial separation of fermented liquor from yeast
may take place by sedimentation under pressure sufficient
to prevent the formation of any gaseous carbon dioxide,
said fermentation and settling zones being separated
one from the other by one or more common walls such
that when fermentation is taking place the pressure
on said common wall(s) in the settling zone is counteracted by the hydrostatic pressure on the common wall(s)
in the fermentation zone, means to allow passage of
liquid from the fermentation zone to the settling zone,
means for discontinuously introducing fermentable liquor to the fermentation zone, an outlet for
separated fermented liquor from the settling zone,
and means for recycling at least part of the
separated yeast from the settling zone to the
fermentation zone.

13. A method of fermentation in which a carbohydrate
solution is fermented to ethanol with a yeast, which
method comprises the steps of
a) passing carbohydrate solution to a fermentation
zone containing yeast whereby the carbohydrate is
fermented to ethanol;
b) allowing the yeast to settle under gravity in the
fermentation zone when fermentation is substantially
complete;
c) subsequently removing a proportion of fermented
liquor substantially free of yeast suspended therein
from the fermentation zone; and
d) replacing the proportion of fermented liquid removed from
the fermentation zone with fresh carbohydrate solution.

**European Patent Office**

**EUROPEAN SEARCH REPORT**

0043259
Application number

EP 81 30 2904

## DOCUMENTS CONSIDERED TO BE RELEVANT

| Category | Citation of document with indication, where appropriate, of relevant passages | Relevant to claim |
|---|---|---|
| | GB - A - 2 021 639 (WAAGNER-BIRO AKTIENGESELLSCHAFT)<br><br> * Claims 1,3,4,8,9,; page 2, line 14,16-17; figure 2 * <br><br>-- | 1,4,6,<br>11 |
| | GB - A - 2 013 716 (ALFA-LAVAL AB)<br><br> * Claims 1,3,4; page 2, lines 12,25-26 * <br><br>-- | 1,4,6,<br>11 |
| P | GB - A - 2 059 988 (A.G. PATENTS LTD.)<br><br> * Claims 1-15 * <br><br>---- | 1,4-13 |

**CLASSIFICATION OF THE APPLICATION (Int. Cl.³)**

C 12 P 7/06

**TECHNICAL FIELDS SEARCHED (Int. Cl.³)**

C 12 P 7/06
C 12 M 1/02

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 03-08-1981 | ALMOND |

EPO Form 1503.1 06.78